(19) Europäisches Patentamt / European Patent Office / Office européen des brevets

(11) EP 1 744 815 B1

(12) **EUROPÄISCHE PATENTSCHRIFT**

(45) Veröffentlichungstag und Bekanntmachung des Hinweises auf die Patenterteilung:
**27.01.2010 Patentblatt 2010/04**

(21) Anmeldenummer: **04821904.2**

(22) Anmeldetag: **03.12.2004**

(51) Int Cl.:
***A61Q 5/10*** *(2006.01)* ***A61K 8/898*** *(2006.01)*

(86) Internationale Anmeldenummer:
**PCT/EP2004/013758**

(87) Internationale Veröffentlichungsnummer:
**WO 2005/102253 (03.11.2005 Gazette 2005/44)**

(54) **HAARFÄRBEMITTEL ENTHALTEND ORGANOSILIKON-COPOLYMERE MIT AMINOGRUPPEN UND POLYOXYALKYLENGRUPPEN SOWIE DEREN VERWENDUNG**

HAIR DYE AGENT COMPRISING ORGANOSILICON COPOLYMERS WITH AMINO GROUPS AND POLYOXYALKYLENE GROUPS AND USE THEREOF

COLORANTS CAPILLAIRES CONTENANT DES COPOLYMERES D'ORGANOSILICONE QUI COMPRENNENT DES GROUPES AMINO ET DES GROUPES POLYOXYALKYLENE, ET LEUR UTILISATION

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **26.04.2004 DE 102004020501**

(43) Veröffentlichungstag der Anmeldung:
**24.01.2007 Patentblatt 2007/04**

(73) Patentinhaber: **Henkel AG & Co. KGaA**
**40589 Düsseldorf (DE)**

(72) Erfinder:
- **KLEEN, Astrid**
  **22763 Hamburg (DE)**
- **ROHLAND, Christa**
  **40468 Düsseldorf (DE)**
- **SCHWARTZ, Stephan**
  **22880 Wedel (DE)**
- **WEISHAUPT, Sarah**
  **20355 Hamburg (DE)**

(56) Entgegenhaltungen:
**EP-A- 1 356 802** **WO-A-99/09939**
**WO-A-2004/064794** **WO-A1-01/41721**
**FR-A- 2 783 416** **GB-A- 2 168 082**
**US-A- 5 910 302**

- **LEIDREITER H I, OESTREICH S: "Kombinationen von aminofunktionellen Siliconderivaten mit kationischen Alkylammoniumverbindungen ergeben ausgewogene Haarbehandlungsmittel" SÖFW - SEIFEN ÖLE FETTE WACHSE, Bd. 10, 2002, Seiten 70-74, XP001205385**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft die Verwendung von Organosilikon-Copolymeren in Mitteln zur oxidativen Färbung keratinischer Fasern, insbesondere menschlicher Haare, derartige Mittel und Verfahren zum Färben keratinischer Fasern, insbesondere menschlicher Haare.

Menschliches Haar wird heute in vielfältiger Weise mit haarkosmetischen Zubereitungen behandelt. Dazu gehören etwa die Reinigung der Haare mit Shampoos, die Pflege und Regeneration mit Spülungen und Kuren sowie das. Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine herausragende Rolle.

Für dauerhafte, intensive Färbungen mit entsprechenden Echtheitseigenschaften werden so genannte Oxidationsfärbemittel verwendet. Solche Färbemittel enthalten üblicherweise Oxidationsfarbstoffvorprodukte, so genannte Entwicklerkomponenten und Kupplerkomponenten. Die Entwicklerkomponenten bilden unter dem Einfluss von Oxidationsmitteln oder von Luftsauerstoff untereinander oder unter Kupplung mit einer oder mehreren Kupplerkomponenten die eigentlichen Farbstoffe aus. Häufig werden auch Kombinationen von Oxidationsfarbstoffen und direktziehenden Farbstoffen zur Erzielung spezieller Nuancen eingesetzt. Die Oxidationsfärbemittel zeichnen sich durch hervorragende, lang anhaltende Färbeergebnisse aus.

Für temporäre Färbungen werden üblicherweise Färbe- oder Tönungsmittel verwendet, die als .färbende Komponente so genannte Direktzieher enthalten. Hierbei handelt es sich um Farbstoffmoleküle, die direkt auf das Haar aufziehen und keinen oxidativen Prozess zur Ausbildung der Farbe benötigen. Zu diesen Farbstoffen gehört beispielsweise das bereits aus dem Altertum zur Färbung von Körper und Haaren bekannte Henna. Diese Färbungen sind gegen Shampoonieren in der Regel empfindlich, so dass eine vielfach unerwünschte Nuancenverschiebung oder gar eine sichtbare "Entfärbung" eintreten kann. Ein Nachteil derartiger temporärer Färbungen liegt darin begründet, dass diese Färbungen sich zu dem natürlichen Haarton hinzu addieren und somit nur Nuancen ermöglichen, die dunkler als der Ausgangston sind. Daher werden auch Färbemittel auf Basis von direktziehenden Farbstoffen häufig in Kombination mit Oxidationsmittelzubereitungen angewendet, um neben der eigentlichen Färbung den Ausgangsfarbton der Fasern aufzuhellen.

Beide Prozesse bedingen daher den Einsatz starker Oxidationsmittel wie beispielsweise Wasserstoffperoxid-Lösungen. Dies kann das zu färbende Haar schädigen. Diesen Schädigungen muss dann mit entsprechenden Pflegeprodukten entgegengewirkt werden.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splissrate verringert.

EP 1 356 802 betrifft Faserbehandlungsmittel, die Pfropfcopolymere enthalten, welche Polyoxyalkylen- und Aminogruppen in den Seitenketten aufweisen und für oxidative Färbung von Haar geeignet sind.

Die WO 99/09939 betrifft ein Haarkonditioniermittel, das unter anderem Polyoxyalkylenamin-Silikon-Copolymere enthält. Die Copolymere sind dabei vorzugsweise Blockcopolymer. In den Beispielen wird unter anderem ein Polyoxyalkylen-Aminosilikon-Copolymer des Typs $(AB)_n$ eingesetzt, das von OSi unter der Bezeichnung Silsoft® A 843 erhältlich ist. In der Schrift wird insbesondere auf Konditioniermittel eingegangen. Daneben wird pauschal auf Dauerwellmittel, Mittel zum Auflösen von Frisuren, Färbe- oder Entfärbemittel hingewiesen. Mittel zur oxidativen Haarfärbung sind nicht erwähnt.

WO 01/41721 betrifft kosmetische Zusammensetzungen; die ein Blockcopolymer des Typs $(AB)_n$ enthalten, wobei A ein Polysiloxanblock und B ein Block ist, der mindestens zwei quartäre Ammoniumgruppen aufweist. Die kosmetischen Zusammensetzungen verleihen Haaren verbesserte kosmetische Eigenschaften wie leichte Kämmbarkeit, Volumen und Glanz. Sie werden insbesondere in Mitteln zum Waschen oder Konditionieren von Haaren eingesetzt. Es wird auch wiederum pauschal auf den Einsatz in Dauerweilmitteln, Mitteln zur Auflösung von Frisuren, Färbe- und Entfärbemitteln hingewiesen. Es ist wiederum kein Hinweis auf Mittel zur oxidativen Haarfärbung enthalten.

Die WO 97/32917 betrifft die Verwendung von Aminosilikon-Polyalkylenoxid-Blockcopolymeren als Weichmacher für insbesondere Textilien. Als, weitere Anwendungsgebiete wird unter anderem die Behandlung von Haaren genannt.

Die Verwendung von Silsoft® A-553 und Silsoft® A-454 von OSi als farberhaltende Konditioniermittel ist ferner in Eurocosmetics 3-2002, Seiten 20 bis 24 beschrieben.

Weiterhin wurden in jüngster Zeit so genannte Kombinationspräparate entwickelt, um den Aufwand der üblichen mehrstufigen Verfahren, insbesondere bei der direkten Anwendung durch den Verbraucher, zu verringern.

Diese Präparate enthalten neben den üblichen Komponenten, beispielsweise zur Färbung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die im Rahmen derartiger Kombinationspräparate einsetzbaren Wirkstoffe müssen insbesondere hinsichtlich

ihrer Stabilität hohen Ansprüchen genügen, da die Färbecremes üblicherweise einen hohen pH-Wert und die Oxidationsmittelzubereitungen einen niedrigen pH-Wert aufweisen. Ferner sind Unverträglichkeiten der verschiedenen Wirkstoffe untereinander und somit eine geringe Lagerstabilität zu vermeiden. Daher sind viele in Nachbehandlungsmitteln verwendete Inhaltsstoffe für Kombinationspräparate ungeeignet.

Es besteht weiterhin ein Bedarf an Wirkstoffen bzw. Wirkstoffkombinationen mit guten pflegenden Eigenschaften, die direkt in Mitteln zur oxidativen Haarfärbung eingesetzt werden können. Ferner besteht weiterhin Nachfrage an Mitteln zur oxidativen Haarfärbung, die eine erhöhte Farbintensität aufweisen.

Aufgabe der vorliegenden Erfindung ist die Bereitstellung von Mitteln zur Verbesserung der Nass und Trokkenkämmbarkeit der Haare sowie der Erhöhung der Farbintensität, die direkt in Mitteln zur oxidativen Haarfärbung mitverwendet werden können. Die Mittel sollen damit für Kombinationspräparate geeignet sein.

Vorzugsweise sollen die Pflegemittel schon in geringen Einsatzmengen eine hohe Wirksamkeit zeigen.

Die Aufgaben werden erfindungsgemäß gelöst durch Verwendung von Organosilikon-Copolymeren, die als Blockcopolymer Polyoxyalkylenblöcke, Polysiloxanblöcke und mindestens zwei in der Polymerhauptkette oder an den Kettenenden kovalent gebundene Aminogruppen enthalten, in Mitteln zur oxidativen Färbung keratinischer Fasern, insbesondere menschlicher Haare.

Es wurde erfindungsgemäß gefunden, dass Organosilikon-Copolymere, die als Blockcopolymer Polyoxyalkylenblöcke, Polysiloxanblöcke und mindestens zwei in der Polymerhauptkette oder an den Kettenenden kovalent gebundene Aminogruppen enthalten, vorteilhaft in Mitteln zur oxidativen Färbung insbesondere menschlicher Haare eingesetzt werden können und bei der Anwendung die Nass- und Trockenkämmbarkeit der Haare verbessern, sowie die Farbintensität der Haarfärbung erhöhen. Die durch den Einsatz der Organosilikon-Copolymere erreichte Pflegewirkung hält auch nach mehreren Haarwäschen an, so dass das Organosilikon-Copolymer eine Depotwirkung auf dem Haar zeigt. Schon in geringen Einsatzkonzentrationen wird eine signifikante Pflegeleistung erreicht.

Die erfindungsgemäß in Mitteln zur oxidativen Färbung eingesetzten Organosilikon-Copolymere sind an sich aus dem Stand der Technik bekannt. Dabei kann beispielsweise auf die vorstehend genannten Schriften verwiesen werden.

Es wurde erfindungsgemäß gefunden, dass die genannten organischen Organosilikon-Copolymere eine hohe Verträglichkeit mit Mitteln zur oxidativen Haarfärbung zeigen und auch unter den Einsatzbedingungen dieser Mittel stabil sind und ihre vorteilhafte Wirkung beibehalten.

Die erfindungsgemäß eingesetzten Organosilikon-Copolymere sind vorzugsweise statistische oder random Blockcopolymere aus Polyoxyalkylenblöcken und Polysiloxanblöcken. Die Anzahl und Länge der Blöcke kann dabei frei gewählt werden. Die Copolymere enthalten mindestens 2, vorzugsweise mindestens 4 in der Polymerhauptkette oder an den Kettenenden kovalent gebundene Aminogruppen.

Vorzugweise enthält das Organosilikon-Copolymer Polyoxyethylen- und Polyoxypropylenblöcke und Polydimethylsiloxanblöcke.

Dabei kann das Organosilikon-Copolymer primäre und/oder sekundäre und/oder tertiäre Aminogruppen aufweisen. Gemäß einer Ausführungsform der Erfindung weist das Blockcopolymer primäre und/oder sekundäre Aminogruppen, besonders bevorzugt primäre und sekundäre Aminogruppen auf. Gemäß einer zweiten Ausführungsform weist das Blockcopolymer tertiäre Aminogruppen auf.

Vorzugsweise weist das Organosilikon-Copolymer Wiederholeinheiten der allgemeinen Formel (I) auf

$$[SiMe_2\text{-}O\text{-}(SiMe_2\text{-}O\text{-})_xSiMe_2\text{-}R\text{-}NH\text{-}R'\text{-}O\text{-}(C_2H_4O)_a\text{-}(C_3H_6O)_b\text{-}R'NH\text{-}R] \qquad (I)$$

mit der Bedeutung

- x Zahl von 3 bis 500,
- a Zahl von 1 bis 300,
- b Zahl von 0 bis 300,
- R unabhängig voneinander zweiwertiger organischer Rest, der über Bindungen Si-C und C-N in die Polymerhauptkette eingebunden ist,
- R' unabhängig voneinander zweiwertiger organischer Rest, der über Bindungen C-O und C-N in die Polymerhauptkette eingebunden ist.

Besonders bevorzugt bedeuten dabei x eine Zahl von 5 bis 300, a eine Zahl von 5 bis 200, b eine Zahl von 4 bis 200, R einen linearen $C_{2-20}$-Alkylenrest und R' einen linearen $C_{2-10}$-Alkylenrest, die jeweils durch eine oder mehrere OH-Gruppen substituiert und durch eine oder mehrere nicht benachbarte Gruppen - O-, -C(O)-, -O-C(O)-, -C(O)-O- unterbrochen sein können.

Besonders bevorzugt ist x eine Zahl von 10 bis 300, a eine Zahl von 5 bis 100, b eine 5 bis 100, R ein linearer $C_{3-10}$-Alkylenrest, der durch mindestens eine OH-Gruppe substituiert und durch mindestens eine Gruppe -O- unterbrochen ist. R' bedeutet besonders bevorzugt einen linearen $C_{1-5}$-Alkylenrest.

Dabei liegen besonders bevorzugt endständig primäre Aminogruppen vor. Besonders bevorzugt ist ein Copolymer, das zwei endständige primäre Aminogruppen und zwei in der Kette befindliche sekundäre Aminogruppen aufweist. Besonders bevorzugt weist das Blockcopolymer auch zusätzlich mindestens 2, besonders bevorzugt genau 2 Hydroxylgruppen auf.

R ist insbesondere ein Ethylen-, Propylen- oder Butylen-Rest, die besonders bevorzugt linear sind, oder es handelt sich um den Rest - $CH_2CH_2CH_2OCH(OH)CH_2$-.

R' ist besonders bevorzugt ein zweiwertiger Alkylenrest wie Ethylen-, Propylen- oder Butylenrest, die insbesondere linear sind.

Besonders bevorzugt wird ein Copolymer der allgemeinen Formel $H_2N(C_3H_6O)(C_2H_4O)CH_2CH_2NHCH_2C(OH)HCH_2$-O-$CH_2CH_2CH_2$-($SiMe_2$)(-O-$SiMe_2$)-$CH_2CH_2CH_2$-O-$CH_2$-C(OH)H-$CH_2NH_2(C_2H_4O)(C_3H_6O)C_3H_6$-$NH_2$.

Die Siloxanblöcke liegen vorzugsweise zu 50 bis 95 mol-%, besonders bevorzugt 70 bis 85 mol-%, bezogen auf das gesamte Blockcopolymer, vor.

Der Amingehalt liegt vorzugsweise im Bereich von 0,02 bis 0,5 meq/g des Copolymers in einer 30% igen Lösung in Dipropylenglykol. Besonders bevorzugt beträgt die Menge 0,05 bis 0,2 meq/g.

Das Molekulargewicht des Blockcopolymer liegt besonders bevorzugt zwischen 5000 und 1000000, insbesondere 10000 bis 200000.

Die Herstellung des Blockcopolymers kann nach bekannten Verfahren erfolgen, beispielsweise durch Umsetzung eines Silikondiepoxides oder eines Dichlorsilikons mit einem Polyoxyalkylendiamin.

Insbesondere wird das unter der Bezeichnung Silsoft® A-843 von OSi, Greenwich, CT, erhältliche Produkt eingesetzt.

Gemäß einer weiteren Ausführungsform der Erfindung weist das Organosilikon-Copolymer tertiäre Aminogruppen auf. Dabei weist das Organosilikon-Copolymer bevorzugt Wiederholeinheiten der allgemeinen Formel (II) auf

$$[(SiMe_2\text{-}O\text{-})_ySiMe_2\text{-}R^1\text{-}]NR^2[R^3\text{-}(OC_2H_4)_c\text{-}(OC_3H_6)_d\text{-}R^4\text{-}NR^5\text{-}]_w \qquad \text{(II)}$$

mit der Bedeutung

| | |
|---|---|
| y | Zahl von 3 bis 500, |
| v | Zahl von 1 bis 50, |
| w | Zahl von 1 bis 50, |
| c | Zahl von 1 bis 300, |
| d | Zahl von 0 bis 300, |
| $R^1$ | unabhängig voneinander zweiwertiger organischer Rest, der über Bindungen Si-C und C-N in die Polymerhauptkette eingebunden ist, |
| $R^3$, $R^4$ | unabhängig voneinander zweiwertige organische Reste, die über Bindungen N-C und C-O in die Polymerhauptkette eingebunden sind, |
| $R^5$ | $C_{1-6}$-Alkyl oder Phenyl, die durch OH substituiert sein können. |

Besonders bevorzugt sind Wiederholeinheiten der allgemeinen Formel (II), in denen y eine Zahl von 5 bis 300, c eine Zahl von 5 bis 200, d eine Zahl von 4 bis 200, $R^1$, $R^3$, $R^4$ unabhängig voneinander lineare $C_{2-20}$-Alkylenreste bedeuten, die jeweils durch eine oder mehrere OH-Gruppen substituiert und durch eine oder mehrere nicht benachbarte Gruppen -O-, -C(O)-, -O-C(O), -C(O)-O- unterbrochen sein können, und $R^2$, $R^5$ unabhängig voneinander $C_{1-4}$-Alkyl bedeuten.

Blockcopolymere der allgemeinen Formel (II) sind beilspielsweise als Silsoft® A-553 von OSi erhältlich.

Die Organosilikon-Copolymere können auch in Form von Umsetzungsprodukten, insbesondere Salzen, von Fettsäuren eingesetzt werden. Beispiele geeigneter Fettsäuren sind $C_{16-30}$-Fettsäuren, ein besonders bevorzugtes Beispiel ist Stearinsäure. Ein derartiges geeignetes Produkt ist Silsoft® A-454 von OSi.

Nach INCI könnte die Bezeichnung Dimethicone Bisamino Hydroxyethyldihydroxypropyl Copolyol/TEA Stearates sein.

In der allgemeine Formel (II) kann $R^1$ dieselbe Bedeutung haben wie R in der allgemeinen Formel (I) $R^3$ kann die gleiche Bedeutung haben wie R' in der allgemeinen Formel (I). Es kann sich dabei insbesondere um Reste handeln, die durch Hydroxylgruppen substituiert und/oder durch Sauerstoffatome unterbrochen sind. Bevorzugt ist eine Kombination aus einer Hydroxylgruppe und einem unterbrechenden Sauerstoffatom, die an nicht benachbarten Kohlenstoffatomen vorliegen.

$R^4$ kann dieselbe Bedeutung wie $R^3$ haben, es kann sich jedoch auch um einen $C_{1-6}$-, vorzugsweise $C_{2-3}$-Alkylenrest handeln.

Geeignete Reste und deren Bedeutungen sind unter anderem in WO 97/32917 angegeben.

Die Herstellung der erfindungsgemäß eingesetzten Organosilikon-Copolymere erfolgt besonders bevorzugt ebenfalls wie in WO 97/32917 beschrieben.

Die erfindungsgemäßen Blockcopolymere werden in den Mitteln zur oxidativen Färbung vorzugsweise in Mengen von 0,1 bis 20 Gew.-%, besonders bevorzugt 0,2 bis 5 Gew.-%, insbesondere 0,3 bis 3 Gew.-%, bezogen auf das gesamte Haarfärbemittel, eingesetzt.

Sie werden dabei insbesondere zur Verbesserung der Nass- und Trockenkämmbarkeit der Haare sowie zur Erhöhung der Farbintensität der Haarfärbemittel eingesetzt.

Erfindungsgemäß besonders bevorzugt wird das Produkt Silsoft® A 843 von OSi eingesetzt.

Die Erfindung betrifft auch ein Mittel zur Färbung keratinischer Fasern, insbesondere menschliche Haare, enthaltend neben Farbstoffvorprodukten mindestens ein Blockcopolymer, wie es vorstehend beschrieben ist. Darüber hinaus enthält das Mittel vorzugsweise mindestens eine Entwicklerkomponente als Farbstoffvorprodukt. Es kann ferner bevorzugt mindestens eine Kupplerkomponente enthalten. Zudem kann es bevorzugt mindestens einen direkt ziehenden Farbstoff enthalten.

Gemäß einer besonders bevorzugten Ausführungsform werden Färbemittel eingesetzt, die keinen Ammoniak enthalten.

Unter keratinischen Fasern werden erfindungsgemäß Pelze, Wolle, Federn und insbesondere menschliche Haare verstanden.

Es wurde erfindungsgemäß gefunden, dass ein Einsatz der Blockcopolymere direkt in Haarfärbemitteln zur oxidativen Haarfärbung nicht nur zu einer signifikanten Verbesserung der Nass- und Trockenkämmbarkeit des Haares direkt nach der Färbung führt, sondern zusätzlich eine Intensivierung der Farbe erzielt wird. Außerdem konnte beobachtet werden, dass die Pflege zahlreiche Shampoowäschen übersteht und damit eine Depotwirkung für das Organosilikon-Copolymer vorliegt.

Weitere wichtige Vorteile des erfindungsgemäß verwendeten Copolymers bei der Formulierung von Haarfärbemitteln sind die gute Wasserlöslichkeit und die Stabilität der Verbindungen in stark alkalischem Medium. Zudem zeigen die Copolymere im Vergleich zu bekannten Mitteln eine geringere Viskositätsveränderung, so dass für die Formulierung von Haarfärbemitteln ein größerer Bereich an Einsatzmengen der Copolymere zugänglich ist. Im Vergleich mit anderen, üblicherweise in Haarfärbemitteln verwendeten Pflegepolymeren zeigten sich für die erfindungsgemäßen Copolymere auch signifikante Pflegeleistungen bei wesentlichen geringeren Einsatzkonzentrationen.

Nachstehend werden die weiteren möglichen Inhaltsstoffe der erfindungsgemäßen Mittel zur oxidativen Haarfärbung näher erläutert.

In einer ersten bevorzugten Ausführungsform enthalten die erfindungsgemäßen Färbemittel mindestens ein Farbstoffvorprodukt. Hinsichtlich der in den erfindungsgemäßen Färbemitteln eingesetzten Farbstoffvorprodukte unterliegt die vorliegende Erfindung keinerlei Einschränkungen. Die erfindungsgemäßen Färbemittel können als Farbstoffvorprodukte

- Oxidationsfarbstoffvorprodukte vom Entwickler- oder Kuppler-Typ, und
- Vorstufen naturanaloger Farbstoffe, wie Indol- und Indolin-Derivate,

sowie Mischungen von Vertretern dieser Gruppen enthalten.

Als Farbstoffvorprodukt enthalten die erfindungsgemäßen Färbemittel bevorzugter Weise mindestens eine Entwicklerkomponente. Als Entwicklerkomponenten werden üblicherweise primäre aromatische Amine mit einer weiteren, in para- oder ortho-Position befindlichen, freien oder substituierten Hydroxy- oder Aminogruppe, Diaminopyridinderivate, heterocyclische Hydrazone, 4-Aminopyrazolderivate sowie 2,4,5,6-Tetraaminopyrimidin und dessen Derivate eingesetzt.

Es kann erfindungsgemäß bevorzugt sein, als Entwicklerkomponente ein p-Phenylendiaminderivat oder eines seiner physiologisch verträglichen Salze einzusetzen.

Erfindungsgemäß ganz besonders bevorzugte p-Phenylendiaminderivate sind p-Phenylendiamin, p-Toluylendiamin, 2-(β-Hydroxyethyl)-p-phenylendiamin und N,N-Bis-(β-hyd roxyethyl)-p-phenylendiamin.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind.

Ganz besonders bevorzugte zweikernige Entwicklerkomponenten sind N,N'-Bis-(β-hydroxyethyl)-N,N'-bis-(4'-aminophenyl)-1,3-diamino-propan-2-ol, Bis-(2-hydroxy-5-aminophenyl)-methan, N,N'-Bis-(4'-aminophenyl)-1,4-diazacycloheptan und 1,10-Bis-(2',5'-diaminophenyl)-1,4,7,10-tetraoxadecan oder eines ihrer physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen.

Ganz besonders bevorzugt sind p-Aminophenol, 4-Amino-3-methylphenol, 4-Amino-2-aminomethylphenol und 4-Amino-2-(diethyl-aminomethyl)-phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise den Pyridin-, Pyrimidin-, Pyrazol-, Pyrazol-Pyrimidin-Derivaten und ihren physiologisch verträglichen Salzen:

Bevorzugte Pyrimidin-Derivate sind insbesondere die Verbindungen, die im deutschen Patent DE 2 359 399, der japanischen Offenlegungsschrift JP 02019576 A2 oder in der Offenlegungsschrift WO 96/15765 beschrieben werden, wie 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Tri-aminopyrimidin.

Bevorzugte Pyrazol-Derivate sind insbesondere die Verbindungen, die in den Patenten DE-A-38 43 892, DE-A-41 33 957 und Patentanmeldungen WO-A-94/08 969, WO-A- 94/08 970, EP-A-740 931 und DE-A-195 43 988 beschrieben werden, wie 4,5-Diamino-1-methylpyrazol, 4,5-Diamino-1-(β-hydroxyethyl)-pyrazol, 3,4-Diaminopyrazol, 4,5-Di-amino-1-(4'-chlorbenzyl)-pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-tert.-butyl-1-methylpyrazol, 4,5-Diamino-1-tert.-butyl-3-methylpyrazol, 4,5-Diamino-1-(β-hydroxy-ethyl)-3-methylpyrazol, 4,5-Diamino-1-ethyl-3-methylpyrazol, 4,5-Diamino-1-ethyl-3-(4'-methoxyphenyl)-pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(□-aminoethyl)-amino-1,3-dimethyl-pyrazol, 3,4,5-Triaminopyrazol, 1-Methyl-3,4,5-triaminopyrazol, 3,5-Diamino-1-methyl-4-methylaminopyrazol und 3,5-Diamino-4-(β-hydroxyethyl)-amino-1-methylpyrazol.

Ferner können die erfindungsgemäßen Mittel kationische Farbstoffvorprodukte vom Kuppler- und/oder Entwicklertyp enthalten, wie sie beispielsweise in den Offenlegungsschriften WO-A1-99/03 819, WO-A2-99/03 834, WO-A1-99/03 836, WO-A1-99148 856, WO-A1-99148 874, WO-A1-99/48 875, WO-A2-00/42 971, WO-A1-00/42 979, WO-A1-00142 980, WO-A1-00/43 356, WO-A1-00/43 367, WO-A1-00/43 368, WO-A1-00/43 386, WO-A1-00/43 388, WO-A1-00/43 389, WO-A1-00/43 396, EP-A1-0 984 006, EP-A1-0 984 007 und EP-A1-0 989 128 beschrieben werden.

Weiterhin sind erfindungsgemäß Färbemittel bevorzugt, die als Farbstoffvorprodukt mindestens eine Kupplerkomponente enthalten. Als Kupplerkomponenten werden in der Regel m-Phenylendiaminderivate, Naphthole, Resorcin und Resorcinderivate, Pyrazolone und m-Aminophenolderivate verwendet. Als Kupplersubstanzen eignen sich insbesondere 1-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 1-Phenyl-3-methylpyrazolon-5, 2,4-Dichlor-3-aminophenol, 1,3-Bis-(2,4-diaminophenoxy)-propan, 2-Chlor-resorcin, 4-Chlor-resorcin, 2-Chlor-6-methyl-3-aminophenol, 2-Amino-3-hydroxypyridin, 2-Methylresorcin, 5-Methylresorcin und 2-Methyl-4-chlor-5-aminophenol.

Besonders bevorzugte Kupplerkomponenten sind 1-Naphthol, 1,5-, 2,7- und 1,7-Dihydroxynaphthalin, 3-Aminophenol, 5-Amino-2-methylphenol, 2-Amino-3-hydroxypyridin, Resorcin, 4-Chlorresorcin, 2-Chlor-6-methyl-3-aminophenol, 2-Methylresorcin, 5-Methylresorcin, 2,5-Dimethylresorcin und 2,6-Dihydroxy-3,4-dimethylpyridin.

Sofern es sich bei den Farbstoffvorprodukten um Amino-Verbindungen handelt, lassen sich aus diesen in üblicher Weise die bekannten Säureadditionssalze herstellen. Alle Aussagen beziehen sich daher sowohl auf die in freier Form vorliegenden Verbindungen als auch auf deren wasserlösliche, physiologischverträgliche Salze. Beispiele für solche Salze sind die Hydrochloride, die Hydrobromide, die Sulfate, die Phosphate, die Acetate, die Propionate, die Citrate und die Lactate.

Die Oxidationsfarbstoffvorprodukte vom Entwickler-/Kupplertyp sind in den erfindungsgemäßen Mitteln bevorzugt in Menge von 0,01 bis 20 Gew.-%, vorzugsweise 0,1 bis 5 Gew.-%, jeweils bezogen auf das gesamte Mittel, enthalten.

Als Farbstoffvorprodukte sind weiterhin Vorstufen naturanaloger Farbstoffe erfindungsgemäß bevorzugt. Als Vorstufen naturanaloger Farbstoffe werden bevorzugt Indole und Indolin eingesetzt, die mindestens eine Hydroxy- oder Aminogruppe, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer zweiten bevorzugten Ausführungsform enthalten die Färbemittel mindestens ein Indol- und/oder Indolinderivat.

Besonders hervorzuheben sind innerhalb dieser Gruppe N-Methyl-5,6-dihydroxyindolin, N-Ethyl-5,6-dihydroxyindolin, N-Propyl-5,6-dihydroxyindolin, N-Butyl-5,6-dihydroxyindolin und insbesondere das 5,6-Dihydroxyindolin.

Innerhalb dieser Gruppe hervorzuheben sind N-Methyl-5,6-dihydroxyindol, N-Ethyl-5,6-dihydroxyindol, N-Propyl-5,6-dihydroxyindol, N-Butyl-5,6-dihydroxyindol sowie insbesondere das 5,6-Dihydroxyindol.

Die Indolin- und Indol-Derivate können in den im Rahmen des erfindungsgemäßen Verfahrens eingesetzten Färbemitteln sowohl als freie Basen als auch in Form ihrer physiologisch verträglichen Salze mit anorganischen oder organischen Säuren, z. B. der Hydrochloride, der Sulfate und Hydrobromide, eingesetzt werden. Die Indol- oder Indolin-Derivate sind in diesen üblicherweise in Mengen von 0,05-10 Gew.-%, vorzugsweise 0,2-5 Gew.-% enthalten.

In einer weiteren Ausführungsform kann es erfindungsgemäß bevorzugt sein, das Indolin- oder Indolderivat in Haarfärbemitteln in Kombination mit mindestens einer Aminosäure oder einem Oligopeptid einzusetzen. Die Aminosäure ist vorteilhafterweise eine α-Aminosäure; ganz besonders bevorzugte α-Aminosäuren sind Arginin, Ornithin, Lysin, Serin

und Histidin, insbesondere Arginin.

In einer zweiten bevorzugten Ausführungsform der vorliegenden Erfindung enthalten die Färbemittel mindestens einen direktziehenden Farbstoff. Dabei spielt es für die erfindungswesentliche Lehre keine Rolle, ob das Färbemittel nur auf direktziehenden Farbstoffen basiert oder ob es diese zur Erzielung der gewünschten färberischen Effekte in Kombination mit den oben genannten Farbstoffvorprodukten enthält.

Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 12, Disperse Blue 3, HC Violet 1, Disperse Violet 1, Disperse Violet 4, Acid Violet 43, Disperse Black 9 und Acid Black 52 bekannten Verbindungen sowie 1,4-Diamino-2-nitrobenzol, 2-Amino-4-nitrophenol, 1,4-Bis-(β-hydroxyethyl)-amino-2-nitrobenzol, 3-Nitro-4-(β-hydroxyethyl)-aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)-amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Hydroxyethyl-2-nitro-toluidin, Pikraminsäure und deren Salze, 2-Amino-6-chloro-4-nitrophenol, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei

(i) kationische Triphenylmethanfarbstoffe, wie beispielsweise Basic Blue 7, Basic Blue 26, Basic Violet 2 und Basic Violet 14,

(ii) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie

(iii) direktziehende Farbstoffe, die einen Heterocyclus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (iii) sind insbesondere die folgenden Verbindungen:

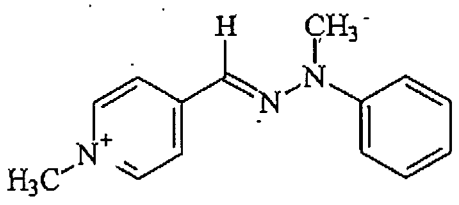

(DZ1)

$CH_3SO_4^-$

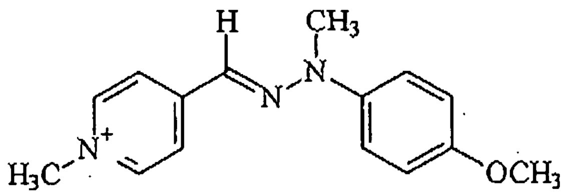

(DZ2)

$Cl^-$

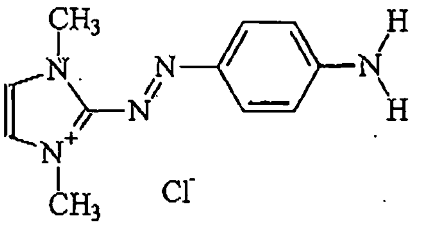

(DZ3)

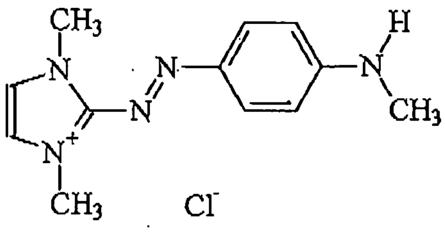

(DZ4)

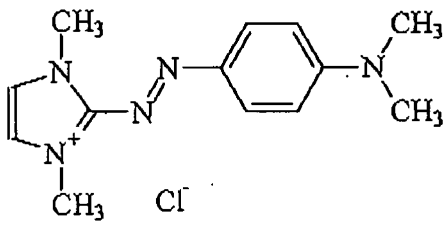

(DZ5)

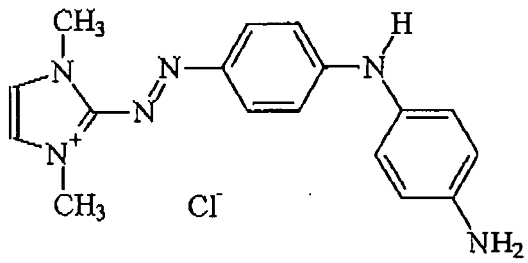

(DZ6)

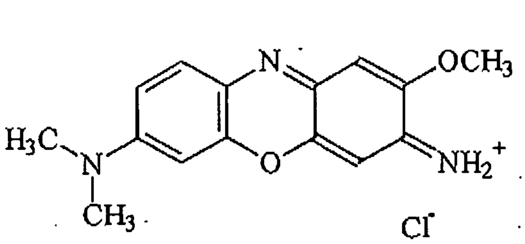

(DZ7)

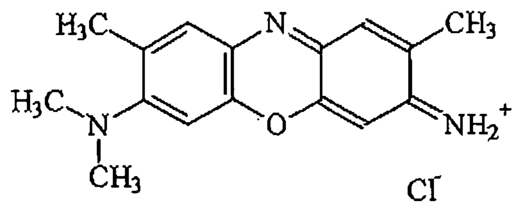

(DZ8)

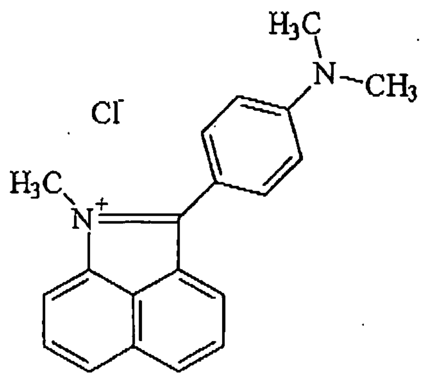

(DZ9)

Die Verbindungen der Formeln (DZ1), (DZ3) und (DZ5) sind ganz besonders bevorzugte kationische direktziehende Farbstoffe der Gruppe (iii).

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Zubereitungen auch in der Natur vorkommende Farbstoffe wie sie beispielsweise in Henna rot, Henna neutral, Henna schwarz, Kamillenblüte, Sandelholz, schwarzen Tee, Faulbaumrinde, Salbei, Blauholz, Krappwurzel, Catechu, Sedre und Alkannawurzel enthalten sind, enthalten.

Es ist nicht erforderlich, dass die Oxidationsfarbstoffvorprodukte oder die direktziehenden Farbstoffe jeweils einheitliche Verbindungen darstellen. Vielmehr können in den erfindungsgemäßen Haarfärbemitteln, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z. B. toxikologischen, ausgeschlossen werden müssen.

Bezüglich der in den erfindungsgemäßen Haarfärbe- und -tönungsmitteln einsetzbaren Farbstoffe wird weiterhin ausdrücklich auf die Monographie Ch. Zviak, The Science of Hair Care, Kapitel 7 (Seiten 248-250; direktziehende Farbstoffe) sowie Kapitel 8, Seiten 264-267; Oxidationsfarbstoffvorprodukte), erschienen als Band 7 der Reihe "Dermatology" (Hrg.: Ch., Culnan und H. Maibach), Verlag Marcel Dekker Inc., New York; Basel, 1986, sowie das "Europäische Inventar der Kosmetik-Rohstoffe", herausgegeben von der Europäischen Gemeinschaft, erhältlich in Diskettenform vom Bundesverband Deutscher Industrie- und Handelsunternehmen für Arzneimittel, Reformwaren und Körperpflegemittel e.V., Mannheim, Bezug genommen.

Haarfärbemittel, insbesondere wenn die Ausfärbung oxidativ, sei es mit Luftsauerstoff oder anderen Oxidationsmitteln wie Wasserstoffperoxid, erfolgt, werden üblicherweise schwach sauer bis alkalisch, d. h. auf pH-Werte im Bereich von etwa 5 bis 11, eingestellt. Zu diesem Zweck enthalten die Färbemittel Alkalisierungsmittel, üblicherweise Alkali- oder Erdalkalihydroxide, Ammoniak oder organische Amine. Bevorzugte Alkalisierungsmittel sind Monoethanolamin, Monoisopropanolamin, 2-Amino-2-methyl-propanol, 2-Amino-2-methyl-1,3-propandiol, 2-Amino-2-ethyl-1,3-propandiol, 2-Amino-2-methylbutanol und Triethanolamin sowie Alkali- und Erdalkalimetallhydroxide. Insbesondere Monoethanolamin, Triethanolamin sowie 2-Amino-2-methyl-propanol und 2-Amino-2-methyl-1,3-propandiol sind im Rahmen dieser Gruppe bevorzugt. Auch die Verwendung von ω-Aminosäuren wie ω-Aminocapronsäure als Alkalisierungsmittel ist möglich. Ammoniak ist ein ganz besonders bevorzugtes Alkalisierungsmittel.

Erfolgt die Ausbildung der eigentlichen Haarfarben im Rahmen eines oxidativen Prozesses, so können übliche Oxidationsmittel wie insbesondere Wasserstoffperoxid oder dessen Anlagerungsprodukte an Harnstoff, Melamin oder Natriumborat verwendet werden. Die Oxidation mit Luftsauerstoff als einzigem Oxidationsmittel kann allerdings bevorzugt sein. Weiterhin ist es möglich, die Oxidation mit Hilfe von Enzymen durchzuführen, wobei die Enzyme sowohl zur

Erzeugung von oxidierenden Per-Verbindungen eingesetzt werden als auch zur Verstärkung der Wirkung einer geringen Menge vorhandener Oxidationsmittel. So können die Enzyme (Enzymklasse 1: Oxidoreduktasen) Elektronen aus geeigneten Entwicklerkomponenten (Reduktionsmittel) auf Luftsauerstoff übertragen. Bevorzugt sind dabei Oxidasen wie Tyrosinase und Laccase aber auch Glucoseoxidase, Uricase oder Pyruvatoxidase. Weiterhin sei das Vorgehen genannt, die Wirkung geringer Mengen (z.B. 1 % und weniger, bezogen auf das gesamte Mittel) Wasserstoffperoxid durch Peroxidasen zu verstärken.

Insbesondere bei schwer färbbarem Haar kann die Zubereitung mit den Farbstoffvorprodukten ohne vorherige Vermischung mit der Oxidationskomponente auf das Haar aufgebracht werden. Nach einer Einwirkdauer von 20 bis 30 Minuten wird dann - gegebenenfalls nach einer Zwischenspülung - die Oxidationskomponente aufgebracht. Nach einer weiteren Einwirkdauer von 10 bis 20 Minuten wird dann gespült und gewünschtenfalls nachshampooniert. Bei dieser Ausführungsform wird gemäß einer ersten Variante, bei der das vorherige Aufbringen der Farbstoffvorprodukte eine bessere Penetration in das Haar bewirken soll, das entsprechende Mittel auf einen pH-Wert von etwa 4 bis 7 eingestellt. Gemäß einer zweiten Variante wird zunächst eine Luftoxidation angestrebt, wobei das aufgebrachte Mittel bevorzugt einen pH-Wert von 7 bis 10 aufweist. Bei der anschließenden beschleunigten Nachoxidation kann die Verwendung von sauer eingestellten Peroxidisulfat-Lösungen als Oxidationsmittel bevorzugt sein.

Unabhängig davon, welches der oben genannten Vorgehen im Rahmen des erfindungsgemäßen Verfahrens gewählt wird, kann die Ausbildung der Färbung dadurch unterstützt und gesteigert werden, dass dem Mittel bestimmte Metallionen zugesetzt werden. Solche Metallionen sind beispielsweise $Zn^{2+}$, $Cu^{2+}$, $Fe^{2+}$, $Fe^{3+}$, $Mn^{2+}$, $Mn^{4+}$, $Li^{+}$, $Mg^{2+}$, $Ca^{2+}$ und $Al^{3+}$. Besonders geeignet sind dabei $Zn^{2+}$, $Cu^{2+}$ und $Mn^{2+}$. Die Metallionen können prinzipiell in der Form eines beliebigen, physiologisch verträglichen Salzes eingesetzt werden. Bevorzugte Salze sind die Acetate, Sulfate, Halogenide, Lactate und Tartrate. Durch Verwendung dieser Metallsalze kann sowohl die Ausbildung der Färbung beschleunigt als auch die Farbnuance gezielt beeinflusst werden.

Unabhängig von der Art des Färbemittels, ist es erfindungsgemäß bevorzugt, das Färbemittel unmittelbar vor der Anwendung mit einer Oxidationsmittelzubereitung zu vermischen.

Ein weiterer Gegenstand der vorliegenden Erfindung ist daher ein Verfahren zur Färbung keratinischer Fasern bei dem eines der erfindungsgemäßen Mittel unmittelbar vor der Anwendung mit einer Oxidationsmittelzubereitung vermischt wird, die resultierende Anwendungszubereitung auf die Fasern aufgetragen wird und nach einer Einwirkungszeit wieder abgespült wird.

In einer weiteren Ausführungsform der vorliegenden Erfindung wird zunächst reine Färbecreme auf das Haar aufgetragen und nach einer Einwirkungszeit ein Mittel, erhältlich durch Mischung der eigentlichen Färbecreme mit der Oxidationsmittelzubereitung.

Ein weiterer Gegenstand der vorliegenden Erfindung ist daher ein Verfahren zum Färben keratinischer Fasern, bei dem eines der erfindungsgemäßen Mittel auf die Fasern aufgetragen wird, nach einer Einwirkungszeit eine zweite Zubereitung, die unmittelbar vor der Anwendung durch Mischung eines der erfindungsgemäßen Mittel mit einer Oxidationsmittelzubereitung erhalten wird, auf die Fasern aufgebracht wird und nach einer Einwirkungszeit die Fasern gründlich gespült werden.

Obwohl prinzipiell alle in der Haarfärbung bekannten Oxidationsmittel eingesetzt werden können, ist Wasserstoffperoxid erfindungsgemäß bevorzugt. Die Oxidationsmittelzubereitung auf Basis des Wasserstoffperoxids weist vorzugsweise einen pH-Wert von 1 bis 6, insbesondere von 2 bis 4, auf. Unmittelbar vor der Anwendung werden üblicherweise die Farbstoff(vorprodukt)zubereitung und die Oxidationsmittelzubereitung in einem Mengenverhältnis von 4:1 bis 1:3, insbesondere von 2:1 bis 1:1, vermischt. Die resultierende Anwendungszubereitung sollte bevorzugt einen pH-Wert im Bereich von 6 bis 12, insbesondere von 9 bis 11, aufweisen. Besonders bevorzugt ist die Anwendung der Haarfärbemittel in einem schwach alkalischen Milieu. Die Anwendungstemperaturen können in einem Bereich zwischen 10 und 60°C, insbesondere zwischen 15 und 40 °C, liegen. Die Anwendung erfolgt bevorzugt bei der Temperatur der Kopfhaut. Zur Verkürzung der Einwirkungszeit beziehungsweise zur Verbesserung des Färbeergebnisses kann eine Anwendung unter Wärmezufuhr, beispielsweise unter einer Wärmehaube erfolgen. Nach einer Einwirkungszeit von ca.. 5 bis 60, insbesondere 15 bis 30, Minuten wird das Haarfärbemittel durch Ausspülen von dem zu färbenden Haar entfernt. Das Nachwaschen mit einem Shampoo entfällt, wenn ein stark tensidhaltiger Träger, z. B. ein Färbeshampoo, verwendet wurde.

In einer bevorzugten Ausführungsform der Erfindung enthält die Anwendungszubereitung mindestens eine quaternäre Ammoniumverbindung. Diese quaternäre Ammoniumverbindung kann erfindungsgemäß Bestandteil der Färbecreme und/oder der Oxidationsmittelzubereitung sein. Es ist aber erfindungsgemäß bevorzugt, dass die quaternäre Ammoniumverbindung Bestandteil der Oxidationsmittelzubereitung ist.

Bevorzugte quaternäre Ammoniumverbindungen sind Ammoniumhalogenide, insbesondere Chloride und Bromide, wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Behenyltrimethylammoniumchlorid, Distearyldimethylammoniumchlorid, Lauryldimethylammoniumchlorid, Lauryldimethylbenzylammoniumchlorid und Tricetylmethylammoniumchlorid, sowie die unter den INCI-Bezeichnungen Quaternium-27 und Quaternium-83 bekannten Imidazolium-Verbindungen. Die langen Alkylketten der oben genannten Tenside weisen bevorzugt 10 bis 18 Kohlenstoffatome auf. Stearyltrimethylammoniumchlorid ist besonders bevorzugt.

Weitere bevorzugte quaternäre Ammoniumverbindungen sind aber auch die so genannten Esterquats. Bei diesen Verbindungen handelt es sich um bekannte Stoffe, die sowohl mindestens eine Esterfunktion als auch mindestens eine quaternäre Ammoniumgruppe als Strukturelement enthalten. Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen. Solche Produkte werden beispielsweise unter den Warenzeichen Stepantex®, Dehyquart® und Armocare® vertrieben. Die Produkte Armocare® VGH-70, ein N,N-Bis(2-Palmitoyloxyethyl)dimethylammoniumchlorid, sowie Dehyquart® F-75, Dehyquart® C-4046, Dehyquart® L80 und Dehyquart® AU-35 sind Beispiele für solche Esterquats.

Weitere erfindungsgemäß bevorzugte quaternäre Ammoniumverbindungen sind die Alkylamidoamine. Die Alkylamidoamine werden üblicherweise durch Amidierung natürlicher oder synthetischer Fettsäuren und Fettsäureschnitte mit Dialkylaminoaminen hergestellt. Eine erfindungsgemäß besonders geeignete Verbindung aus dieser Substanzgruppe stellt das unter der Bezeichnung Tegoamid® S 18 im Handel erhältliche Stearamidopropyl-dimethylamin dar.

Ein weiterer Gegenstand der vorliegenden Erfindung ist ein 2-Komponenten-Kit zur Färbung keratinischer Fasern, umfassend eine erste Zubereitung wie vorstehend beschrieben sowie eine zweite Zubereitung, enthaltend mindestens ein Oxidationsmittel und mindestens eine quaternäre Ammoniumverbindung.

Im Bezug auf die im Rahmen dieses Gegenstandes einsetzbaren Oxidationsmittel und quaternären Ammoniumverbindungen sei auf die obigen Ausführungen verwiesen.

Die erfindungsgemäßen Mittel können weiterhin alle für solche Zubereitungen bekannten Wirk-, Zusatz- und Hilfsstoffe enthalten. In vielen Fällen enthalten diese Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslichmachende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein.

Ferner können die erfindungsgemäßen Mittel bevorzugt noch einen weiteren konditionierenden Wirkstoff, ausgewählt aus der Gruppe, die von kationischen Tensiden, kationischen Polymeren, Alkylamidoaminen, Paraffinölen, pflanzlichen Ölen und synthetischen Ölen gebildet wird, enthalten.

Als konditionierende Wirkstoffe bevorzugt sein können kationische Polymere. Dies sind in der Regel Polymere, die ein quartäres Stickstoffatom, beispielsweise in Form einer Ammoniumgruppe, enthalten.
Bevorzugte kationische Polymere sind beispielsweise

- quaternisierte Cellulose-Derivate, wie sie unter den Bezeichnungen Celquat® und Polymer JR® im Handel erhältlich sind. Die Verbindungen Celquat® H 100, Celquat® L 200 und Polymer JR®400 sind bevorzugte quaternierte Cellulose-Derivate.
- Copolymere des Vinylpyrrolidons mit quaternierten Derivaten des Dialkylaminoacrylats und -methacrylats, wie beispielsweise mit Diethylsulfat quaternierte Vinylpyrrolidon-Dimethylaminomethacrylat-Copolymere. Solche Verbindungen sind unter den Bezeichnungen Gafquat®734 und Gafquat®755 im Handel erhältlich.
- Vinylpyrrolidon-Methoimidazoliniumchlorid-Copolymere, wie sie unter der Bezeichnung Luviquat® angeboten werden.
- quaternierter Polyvinylalkohol
  sowie die unter den Bezeichnungen
- Polyquaternium-2,
- Polyquaternium-17,
- Pofyquaternium-18 und
- Polyquaternium-27 bekannten Polymeren mit quartären Stickstoffatomen in der Polymerhauptkette.

Besonders bevorzugt sind kationische Polymere der vier erstgenannten Gruppen, ganz besonders bevorzugt sind Polyquaternium-2, Polyquaternium-10 und Polyquaternium-22.

Ebenfalls einsetzbar als konditionierende Wirkstoffe sind Paraffinöle, synthetisch hergestellte oligomere Alkene sowie pflanzliche Öle wie Jojobaöl, Sonnenblumenöl, Orangenöl, Mandelöl, Weizenkeimöl und Pfirsichkernöl.

Gleichfalls geeignete haarkonditionierende Verbindungen sind Phospholipide, beispielsweise Sojalecithin, Ei-Lecithin und Kephaline sowie die unter den INCI-Bezeichnungen Linoleamidopropyl PG-Dimonium Chloride Phosphate, Cocamidopropyl PG-Dimonium Chloride Phosphate und Stearamidopropyl PG-Dimonium Chloride Phosphate bekannten Substanzen. Diese werden beispielsweise von der Firma Mona unter den Handelsbezeichnungen Phospholipid EFA®, Phospholipid PTC® sowie Phospholipid SV® vertrieben.

Weitere Wirk-, Hilfs- und Zusatzstoffe sind beispielsweise

- nichtionische Polymere wie beispielsweise VinylpyrrolidonNinylacrylat-Copolymere, Polyvinylpyrrolidon und Vinylpyrrolidon/Vinylacetat-Copolymere und Polysiloxane,
- zwitterionische und amphotere Polymere wie beispielsweise Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere,
- anionische Polymere wie beispielsweise Polyacrylsäuren, vernetzte Polyacrylsäuren, Vinylacetat/Crotonsäure-Copolymere, Vinylpyrrolidon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert.Butyl-acrylamid-Terpolymere,
- Verdickungsmittel wie Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol,
- Strukturanten wie Maleinsäure und Milchsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsmittel und -vermittler wie Ethanol, Isopropanol, Ethylenglykol, Propylenglykol, Glycerin und Diethylenglykol,
- faserstrukturverbessernde Wirkstoffe, insbesondere Mono-, Di- und Oligosaccharide wie beispielsweise Glucose, Galactose, Fructose, Fruchtzucker und Lactose,
- quaternierte Amine wie Methyl-1-alkylamidoethyl-2-alkylimidazolinium-methosulfat
- Entschäumer wie Silikone,
- Farbstoffe zum Anfärben des Mittels,
- Antischuppenwirkstoffe wie Piroctone Olamine, Zink Omadine und Climbazol,
- Lichtschutzmittel, insbesondere derivatisierte Benzophenone, Zimtsäure-Derivate und Triazine,
- Substanzen zur Einstellung des pH-Wertes, wie beispielsweise übliche Säuren, insbesondere Genußsäuren und Basen,
- Wirkstoffe wie Allantoin, Pyrrolidoncarbonsäuren und deren Salze sowie Bisabolol,
- Vitamine, Provitamine und Vitaminvorstufen, insbesondere solche der Gruppen A, $B_3$, $B_5$, $B_6$, C, E, F und H,
- Pflanzenextrakte wie die Extrakte aus Grünem Tee, Eichenrinde, Brennessel, Hamamelis, Hopfen, Kamille, Klettenwurzel, Schachtelhalm, Weißdorn, Lindenblüten, Mandel, Aloe Vera, Fichtennadel, Roßkastanie, Sandelholz, Wacholder, Kokosnuß, Mango, Aprikose, Limone, Weizen, Kiwi, Melone, Orange, Grapefruit, Salbei, Rosmarin, Birke, Malve, Wiesenschaumkraut, Quendel, Schafgarbe, Thymian, Melisse, Hauhechel, Huflattich, Eibisch, Meristem, Ginseng und Ingwerwurzel,
- Cholesterin,
- Konsistenzgaber wie Zuckerester, Polyolester oder Polyolalkylether,
- Fette und Wachse wie Walrat, Bienenwachs, Montanwachs und Paraffine,
- Fettsäurealkanolamide,
- Komplexbildner wie EDTA, NTA, β-Alanindiessigsäure und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex, Styrol/PVP- und Styrol/Acrylamid-Copolymere
- Perlglanzmittel wie Ethylenglykolmono- und -distearat sowie PEG-3-distearat,
- Pigmente,
- Stabilisierungsmittel für Wassserstoffperoxid und andere Oxidationsmittel,
- Treibmittel wie Propan-Butan-Gemische, $N_2O$, Dimethylether, $CO_2$ und Luft,
- Antioxidantien.

Bezüglich weiterer fakultativer Komponenten sowie die eingesetzten Mengen dieser Komponenten wird ausdrücklich auf die dem Fachmann bekannten einschlägigen Handbücher, z. B. Kh. Schrader, Grundlagen und Rezepturen der Kosmetika, 2. Auflage, Hüthig Buch Verlag, Heidelberg, 1989, verwiesen.

Die folgenden Beispiele sollen den Gegenstand der vorliegenden Anmeldung näher erläutern.

**Beispiele**

Es wurden folgende Färbemittel hergestellt (alle Mengenangaben sind, soweit nicht anders vermerkt, Gewichtsteile)

**1. Färbecreme A - D**

| | |
|---|---|
| Fettalkoholgemisch $C_{12}$-$C_{18}$ | 7,5 |
| Eutanol G[5] | 1,0 |
| Eumulgin® B1[4] | 0,5 |
| Eumulgin® B2[1] | 0,4 |
| Plantacare® 1200[2] | 3,0 |
| Dehyton® K[3] | 2,5 |
| Ammoniumsulfat | 0,5 |
| Natriumsulfit | 0,8 |
| Farbstoffgemisch a - d | wie in Tabelle 1 angegeben |
| Ammoniak (25-%ig in Wasser) | ad pH 10 |
| Wasser | ad 100 |

[1] Cetearylalkohol mit ca. 20 EO-Einheiten (INCI-Bezeichnung: Ceteareth-20) (COGNIS)
[2] C12-16-Fettalkohol-1,4-glucosid (ca. 50% Aktivsubstanz; INCI-Bezeichnung: Lauryl Glucoside) (COGNIS)
[3] Fettsäureamid mit Betainstruktur (ca. 30 % Aktivsubstanz; INCI-Bezeichnung: Cocamidopropyl Betaine) (Henkel)
[4] Cetylstearylalkohol + 12 EO (INCI-Bezeichnung: Ceteareth-12) (COGNIS)
[5] 2-Octyldodecylfettalkohol, INCI-Bezeichnung: Octyldodecanol (COGNIS)

Tabelle 1:

| Farbstoffgemisch | a | b | c | d |
|---|---|---|---|---|
| PTD Sulfat | 0.3 | 0.2 | 0.17 | 0.4 |
| TAP Sulfat | | | | 1.6 |
| Oxyrot | 0.9 | 0.36 | | 0.05 |
| Resorcin | | | | 0.1 |
| 2-Methylresorcin | | | 0.03 | 1.0 |
| 4-Chlorresorcin | 0.08 | | 0.12 | |
| m-Aminophenol | | | 0.03 | |
| Methylgelb | 0.1 | | | |
| p-Amino-o-kresol | 0.9 | 0.1 | | |
| 2,7-Dihydroxynaphthalin | | 0.17 | | |
| 6-Chlor-4-nitro-2-aminophenol | | 0.1 | | |

Zur Ausfärbung wurde jeweils 1 Teil der oben beschriebenen Färbemittel mit 1 Teil einer 6 %igen Wasserstoffperoxidlösung vermischt. Gegebenenfalls wurde zuvor das zu untersuchende Pflegepolymer (I = Silsoft® A843, II = Polymer JR 400 von Amerchol, III = Silsoft® A454, IV = Polymer W 37194 von Stockhausen) eingesetzt. Die Pflegepolymere II und IV sind dabei Vergleichsprodukte aus dem Stand der Technik. Beim Polymer W 37194 von Stockhausen

handelt es sich um ein Copolymerisat von Acrylsäure-Natriumsalz und Acrylamidopropyltrimethylammoniumchlorid in Wasser. Beim Polymer JR 400 von Amerchol handelt es sich um eine quaternierte Hydroxyethylcellulose, nach INCI als Polyquaternium-10 bezeichnet.

Insbesondere das Polymer W 37194 führt zu Viskositätsproblemen beim Einsatz in höheren Konzentrationen.

Diese Anwendungszubereitung wurde auf naturweißes Humanhaar (von Alkinco) im Flottenverhältnis 1 : 4 aufgebracht, dort für 30 Minuten belassen und dann ausgespült. Die Ergebnisse der Ausfärbungen wurden farbmetrisch untersucht und sind nachfolgend zusammengestellt:

| **Färbemittel** | **A** | **B** | **C** | **D** |
|---|---|---|---|---|
| Färbeergebnis | kupferrot | kupfergold | braun | rot |

Es wurde farbmetrisch nach CieLAB die Buntheit C* bestimmt, vergleiche WO 01/21145. Nach der Regel ΔC* (Buntheitsdifferenz) = C* Probe -C* Standard bedeutet dies bei ΔC* positiv, dass das Muster reiner in der Farbe ist. Die erhaltenen Werte belegen, dass durch Zusatz von Polymer eine reinere Farbe erzielt wird. Am vorteilhaftesten ist der Zusatz von Polymer I in 0.5 % Konzentration, gefolgt von Polymer III:

| **Färbemittel** | **A** | **B** | **C** | **D** |
|---|---|---|---|---|
| C* (ohne Polymer) | 24.69 | 27.36 | 14.37 | 26.44 |
| C* (mit 0.75% AS I) | - | - | **17.12** | - |
| C* (mit 0.5.% AS I) | **27.98** - | **29.64** | 16.78 | **29.25** |
| C* (mit 0.75 % AS II) | 26.25 | 29.52 | 16.16 | 27.94 |
| C* (mit 0.5 % AS II) | 26.83 | 28.55 | 16.15 | 27.82 |
| C* (mit 0,75% AS III) | - | - | 16.88 | 28.03 |
| C* (mit 0.5 % AS III) | - | - | 16.97 | 28.87 |
| C* (mit 0,75 % ASII) | - | - | 16.55 | 28.03 |

Es wurden weiterhin die a*-Werte bestimmt. Nach der Formel Δa* (Farbdifferenz an der rot/grün-Achse) = a* Probe - a* Muster gilt, dass bei Δa* positiv das Muster roter ist. Die erhaltenen Werte zeigen, dass durch Zusatz von Polymer für die hier überwiegend ausgewählten Rotnuancen ein roteres Farbergebnis erzielt wird. Auch hier zeigt sich das Polymer I, insbesondere in 0.5 % Konzentration, als bestes Additiv, Polymer III als zweitbestes Additiv.

| **Färbemittel** | **A** | **B** | **C** | **D** |
|---|---|---|---|---|
| A* (ohne Polymer) | 21.94 | 16.24 | 4.76 | 23.79 |
| a* (mit 0.75 % AS I) | **23.38** | - | 5.26 | 24.87 |
| a* (mit 0,5 % AS I) | 23.06 | **17.39** | **5.44** | **24.99** |
| a* (mit 0,75 % AS II) | 21.81 | 17.11 | 5.03 | 23.73 |
| a* (mit 0,5 % ASII) | 21.75 | 17.03 | 5.11 | 23.71 |
| a* (mit 0,75 % AS III) | 22.76 | - | - | 24.85 |
| a* (mit 0.5 % AS III) | 22.98 | - | 5.33 | 24.88 |
| a* (mit 0.75 % AS IV) | 22.55 | - | 5.18 | 23.99 |

Des Weiteren wurde die Waschbeständigkeit der Nuancen auf normal bewittertem Haar geprüft. Farbmetrisch wurde die Rest-Farbintensität (Farbstärke) F [%] im Vergleich zur jeweils ungewaschenen Strähne (= 100 % Farbintensität) bestimmt. Die Färbung der Strähnen wurde farbmetrisch an 4 Meßpunkten mit dem Gerät Datacolor Text Flash, der Firma Data Color International vermessen, die Messergebnisse mit der Software Data Color Tools QC gemäß Formel (I) ausgewertet und in den folgenden Tabellen zusammengefasst. Als Referenz diente die Färbung einer ungewaschenen Strähne

$$\frac{K/S_{Probe}}{K/S_{Referenz}} * 100 = Farbstärke[\%]$$

mit

K = Absorptionskoeffizient
S = Struekoeffizient
K/S = Reflektiönskoeffizient

Die Messergebnisse zeigen deutlich, dass durch Zusatz von Polymer eine bessere Waschbeständigkeit erzielt wird. Am vorteilhaftesten ist hier das Polymer III, gefolgt von Polymer I:

| Färbemittel | A | B | C | D |
|---|---|---|---|---|
| F (ohne Polymer) | 82.3 | 75.8 | 79.3 | 85.4 |
| F (mit 0.75% AS I) | - | 78.9 | **91.8** | **97.5** |
| F (mit 0,5 % AS I) | **88.0** | **82.9** | 91.2 | 91.6 |
| F (mit 0.75 % AS II) | 84.2 | 75.1 | 88.7 | 92.9 |
| F (mit 0,5 % AS II) | 83.9 | 77.2 | 82.5 | 89.3 |
| F (mit 0.75 % AS III) | 89.9 | 85.7 | 95.3 | 97.4 |
| F (mit 0,5 % AS III) | 89.5 | 84.9 | 94.9 | 97.0 |
| F (mit 0.75 % AS IV) | 85.3 | 79.9 | - | 90.5 |

Weiterhin wurde die Waschbeständigkeit der Nuancen auf stark strapaziertem Haar geprüft. Farbmetrisch wurde die Rest-Farbintensität (Färbestärke) F [%] im Vergleich zur jeweils ungewaschenen Strähne (= 100 % Farbintensität) bestimmt. Die Messergebnisse zeigen deutlich, dass durch Zusatz von..Polymer eine bessere Waschbeständigkeit erzielt wird. Am vorteilhaftesten ist wiederum das Polymer III:

| **Färbemittel** | **A** | **B** | **C** | **D** |
|---|---|---|---|---|
| F (ohne Polymer) | 66.6 | - | 62.4 | |
| F (mit 0.75 % ASI), | - | - | - | - |
| F (mit 0.5 % AS I) | **84.9** | - | **70.7** | - |
| F (mit 0.75 % AS II) | 79.2 | - | 68.0 | - |
| F (mit 0.5 % AS II) | 72.3 | - | 63.5 | - |
| F (mit 0.75 % AS III) | 82.3 | - | 72.5 | 93.7 |
| F (mit 0.5 % AS III) | 85.7 | - | 78.1 | 93.8 |
| F (mit 0.75% AS IV) | 82.9 | - | 70.2 | 90.8 |

Als weiterer Parameter wurde die Avivageleistung der Polymere im Vergleich getestet (in Färbemittel C). Es wurde im Testsalon an 5 Modellen geprüft. Dabei wurde die Avivage nach Schulnotensystem beurteilt:

| Polymer | Modell 1 | Modell 2 | Modell 3 | Modell 4 | Modell 5 |
|---|---|---|---|---|---|
| I, 0.5 % | 1 | 1 | 1.5 | 1 | 1 |
| I, 0.75 % | 1 | 1 | 1 | 1 | 1 |
| II, 0.5 % | 2.5 | 2 | 3 | 2.5 | 2.5 |
| II, 0.75 % | 2.5 | 2 | 2.5 | 2.5 | 2.5 |
| III, 0.5 % | 2 | 1.5 | 2 | 2 | 2 |

(fortgesetzt)

| Polymer | Modell 1 | Modell 2 | Modell 3 | Modell 4 | Modell 5 |
|---|---|---|---|---|---|
| III, 0.75 % | 1.5 | 1 | 2 | 1.5 | 1.5 |
| ohne | 4 | 3.5 | 4 | 3 | 4.5 |
| Ergebnis: Polymer I und III sind auch in geringerer Konzentration als II besser in der Avivage. | | | | | |

Die erfindungsgemäß eingesetzten Organosiloxan-Copolymere zeigen im Vergleich zu den Vergleichspolymeren eine konstante Viskosität, unabhängig vom Elektrolytgehalt. Zudem zeigen die Cremeemulsionen, die die erfindungsgemäßen Organosiloxan-Copolymere enthalten, eine bessere Konsistenz.

Im Vergleich zu Polymer JR 400 liefert Silsoft® A-843 bereits in wesentlich geringeren Einsatzkonzentrationen eine signifikante Pflegeleistung, die mit steigender Menge zunimmt. Im Vergleich um Polymer W 37194 kann eine verbesserte Farb- und Pflegeleistung der Formulierung bei geringerer Einsatzkonzentration erzielt werden.

2. Färbecreme E

| | |
|---|---|
| 50%ige KOH | 2,45 |
| Carbomer[6] 934 | 0,15 |
| $TiO_2$ | 0,5 |
| Polyethylenglykol 600 | 0,6 |
| Laurethsulfat Na, 27%ig | 4,4 |
| Lanette[7] E | 0,5 |
| Kaliumoleinseife, 12,5%ig | 3,0 |
| Emulgin B2 | 3,0 |
| Eutanol G. | 2,0 |
| Cetyl/Stearylalkohol 50:50 | 10,2 |
| Cutina[8] AGS | 2,0 |
| Cutina[9] GMS-SE | 1,0 |
| Farbstoffgemisch | 5,6 |
| Tetranatrium EDTA | 0,1 |
| Tetranatrium EDTA | 0,2 |
| Silsoft® A-843 | 0,5 |
| Merquat[11] Plus 3330 | 1,5 |
| Ascorbinsäure | 0,05 |
| Natriumsulfit | 0,2 |
| Monoethanolamin | 3,0 |
| Essential Oil 14213 | 0,14 |
| Wasser | ad 100 |

Farbstoffgemisch:

| | |
|---|---|
| Hochdisperse Kieselsäure | 0,25 |
| p-Toluoldiaminsulfat | 3,24 |
| Resorcin | 0,81 |
| m-Aminophenol | 0,16 |
| 4-Chlvrresorcin | 0,57 |
| 2,4-Diaminophenoxyethanol 2 HCl | 0.58 |
| - | 5,6 |

Entwickler F

| | |
|---|---|
| Dipicolinsäure | 0,1 |
| 50%ige KOH | 0,3 |
| Natriumbenzoat | 0,04 |
| Natriumpyrophosphat | 0,1 |
| Turpinal[12] SL | 0,4 |
| 1,2-Propylenglykol | 0,4 |
| Cetyl/Stearylalkohol 50 : 50 | 4,0 |
| Dehyquart[13] B | 0,75 |
| Emulgin B2 | 1,2 |
| Paraffinöl | 0,3 |
| 50%ige $H_2O_2$ | 6,2 bzw. 12,2 bzw. 8,4 |

Färbecreme und Entwickler (mit 4 % $H_2O_2$) werden im Gewichtsverhältnis 1 : 2 bei der Anwendung gemischt. Die Einwirkzeit beträgt etwa 20 Minuten.

| | |
|---|---|
| 6 | Acrylsäure quervernetzt mit Polyalkylenpolyether (INCI-Bezeichnung-. Carbomer) (Noveon) |
| 7 | Fettalkoholsulfat-Natrium-Salz (INCI-Bezeichnung: Sodium Cetearyl Sulfate) (Henkel) |
| 8 | Ethylenglykoldistearat (Cognis) |
| 9 | Glycerylstearat (INCI-Bezeichnung: Olyceryl Stearate SE) (Cognis) |
| 10 | INCI-Bezeichnung: Linoleamidopropyl-PG-dimoniumchloridphosphate (Uniqema) |
| 11 | Dimethyldialkylammoniumchlorid-Acrylsäure-Acrylamid-Terpolymer (INCI-Bezeichnung: Polyquaternium-39) (Ondeo-Nalco) |
| 12 | 1-Hydroxyethan-1,1-diphosphonsäure (HEDP) (INCI-Bezeichnung: Etidromic Acid, Aqua) (Solutia) |
| 13 | Stearyltrimethylammoniumchlorid (INCI-Bezeichnung: Steartrimonium Chloride) (Cognis |

## Patentansprüche

1. Verwendung von Organosilikon-Copolymeren, die als Blockcopolymer Polyoxyalkylenblöcke, Polysiloxanblöcke und mindestens zwei in der Polymerhauptkette oder an den Kettenenden kovalent gebundene Aminogruppen enthalten, in Mitteln zur oxidativen Färbung keratinischer Fasern, insbesondere menschlicher Haare.

2. Verwendung nach Anspruch 1, **dadurch gekennzeichnet, dass** das Organosiloxan-Copolymer ein statistisches oder random Blockcopolymer aus Polyoxyalkylenblöcken und Polysiloxanblöcken ist.

3. Verwendung nach Anspruch 1 oder 2, **dadurch gekennzeichnet, dass** das Organosilikon-Copolymer Polyoxyethylen- und Polyoxypropylenblöcke und Polydimethylsiloxanblöcke enthält.

4. Verwendung nach einem der Ansprüche 1 bis 3, **dadurch gekennzeichnet, dass** das Organosilikon-Copolymer primäre und/oder sekundäre Aminogruppen aufweist.

5. Verwendung nach einem der Ansprüche 1 bis 4, **dadurch gekennzeichnet, dass** das Organosilikon-Copolymer Wiederholeinheiten der allgemeinen Formel (I) aufweist

$$[SiMe_2\text{-}O\text{-}(SiMe_2\text{-}O\text{-})_xSiMe_2\text{-}R\text{-}NH\text{-}R'\text{-}O\text{-}(C_2H_4O)_a\text{-}(C_3H_6O)_b\text{-}R'NH\text{-}R] \qquad (I)$$

mit der Bedeutung

x Zahl von 3 bis 500,

a Zahl von 1 bis 300,
b Zahl von 0 bis 300,
R unabhängig voneinander zweiwertiger organischer Rest, der über Bindungen Si-C und C-N in die Polymerhauptkette eingebunden ist,
R' unabhängig voneinander zweiwertiger organischer Rest, der über Bindungen C-O und C-N in die Polymerhauptkette eingebunden ist.

**6.** Verwendung nach einem der Ansprüche 4 oder 5, **dadurch gekennzeichnet, dass** das Organosilikon-Copolymer endständig primäre Aminogruppen aufweist.

**7.** Verwendung nach einem der Ansprüche 1 bis 3, **dadurch gekennzeichnet, dass** das Organositikon-Copolymer tertiäre Aminogruppen aufweist.

**8.** Verwendung nach Anspruch 7, **dadurch gekennzeichnet, dass** das Organosilikon-Copolymer Wiederholeinheiten der allgemeinen Formel (II) aufweist

$$[(SiMe_2\text{-}O\text{-})_ySiMe_2\text{-}R^1\text{-}]_yNR^2[R^3\text{-}(OC_2H_4)_c\text{-}(OC_3H_6)_d\text{-}R^4\text{-}NR^5\text{-}]_w \qquad (II)$$

mit der Bedeutung

y Zahl von 5 bis 300,
v Zahl von 1 bis 50,
w Zahl von 1 bis 50,
c Zahl von 5 bis 200,
d Zahl von 4 bis 200,
$R^1$, $R^3$, $R^4$ unabhängig voneinander lineare $C_{2-20}$-Alkylenreste, die jeweils durch eine oder mehrere OH-Gruppen substituiert und durch eine oder mehrere nicht benachbarte Gruppen -O-, -C(O)-, -O-C(O), -C(O)-O- unterbrochen sein können, und
$R^2$, $R^5$ unabhängig voneinander $C_{1-4}$-Alkyl.

**9.** Verwendung nach einem der Ansprüche 7 bis 8, **dadurch gekennzeichnet, dass** das Organosilikon-Copolymer mit einer $C_{16-24}$-Fettsäure umgesetzt ist.

**10.** Verwendung nach einem der Ansprüche 1 bis 9, wobei die Organosilikon-Copolymere zur Verbesserung der Nass- und Trockenkämmbarkeit der Haare dienen.

**11.** Verwendung nach einem der Ansprüche 1 bis 9, **dadurch gekennzeichnet, dass** die Organosilikon-Copolymere zur Erhöhung der Farbintensität dienen.

**12.** Mittel zur oxidativen Färbung keratinischer Fasern, insbesondere menschlicher Haare, enthaltend neben Farbstoffvorprodukten Organosilikon-Copolymere, wie sie in einem der Ansprüche 1 bis 9 definiert sind.

**13.** Verfahren zum Färben keratinischer Fasern, insbesondere menschlicher Haare, **dadurch gekennzeichnet, dass** ein Mittel nach Anspruch 12 unmittelbar vor der Anwendung mit einer Oxidationsmittelzubereitung vermischt wird, die resultierende Anwendungszubereitung auf die Fasern aufgebracht wird und nach einer Einwirkungszeit wieder abgespült wird.

**14.** Verfahren zum Färben keratinischer Fasern, insbesondere menschlicher Haare, **dadurch gekennzeichnet, dass** ein Mittel nach Anspruch 12 auf die Fasern aufgetragen wird, nach einer Einwirkungszeit eine zweite Zubereitung, die unmittelbar vor der Anwendung durch Mischen eines Mittels nach Anspruch 12 mit einer Oxidationsmittelzubereitung erhalten wird, auf die Fasern aufgebracht wird und nach einer Einwirkungszeit die Fasern gründlich gespült werden.

**15.** Zwei-Komponenten-Kit zur Färbung keratinischer Fasern, insbesondere menschlicher Haare, umfassend eine erste Zubereitung nach Anspruch 12 sowie eine zweite Zubereitung, enthaltend mindestens ein Oxidationsmittel.

## Claims

1. Use of organosilicone copolymers, which contain, as block copolymers, polyoxyalkylene blocks, polysiloxane blocks and at least two amino groups, which are covalently linked to the main polymer chain or to the chain ends, in agents for the oxidative dyeing of keratinic fibers, especially of human hair.

2. The use according to claim 1, **characterized in that** the organosiloxane copolymer is a random block copolymer of polyethylene oxide and polypropylene oxide blocks and polysiloxane blocks.

3. The use according to claims 1 or 2, **characterized in that** the organosilicone copolymer contains polyethylene oxide and polypropylene oxide blocks and polydimethylsiloxane blocks.

4. Use according to one of the claims 1 to 3, **characterized in that** the organosilicone copolymer has primary and/or secondary amino groups.

5. The use according to one of the claims 1 to 4, **characterized in that** the organosilicone copolymer has repeating units of the general formula (I)

$$[SiMe_2\text{-O-}(SiMe_2\text{-O-})_xSiMe_2\text{-R-NH-R'-O-}(C_2H_4O)_a\text{-}(C_3H_6O)_b\text{-R'NH-R}] \qquad (I)$$

in which

x is a number from 3 to 500
a is a number from 1 to 300
b is a number from 0 to 300
R independently of one another represents a bivalent organic group, which is tied into the main polymer chain over Si-C and C-N bonds,
R' independently of one another represents a bivalent organic group, which is tied into the main polymer chain over C-O and C-N bonds,

6. The use according to one of the claims 4 or 5, **characterized in that** the organosilicone copolymer has a terminal primary amino group.

7. The use according to one of the claims 1 to 3, **characterized in that** the organosilicone copolymer has tertiary amino groups.

8. The use according to claim 7, **characterized in that** the organosilicone copolymer has repeating units of the general Formula (II)

$$[(SiMe_2\text{-O-})_ySiMe_2\text{-}R^1\text{-}]_vNR^2[R^3\text{-}(OC_2H_4)_c\text{-}(OC_3H_6)_d\text{-}R^4\text{-}NR^5\text{-}]_w \qquad (II)$$

in which

y represents a number from 5 to 300,
v represents a number from 1 to 50,
w represents a number from 1 to 50,
c represents a number from 5 to 200,
d represents a number from 4 to 200,

$R^1$, $R^3$, $R^4$, independently of one another, represent linear $C_{2-20}$ alkylene groups, which in each case may be substituted by one or more OH and interrupted by one or more nonadjacent -O-, -C(O)-, -O-C(O)-, -C(O)-O-groups, and $R^2$, $R^5$, independently of one another represent $C_{1-4}$ alkyl groups.

9. The use according to one of the claims 7 to 8, **characterized in that** the organosilicone copolymer is reacted with a $C_{16-24}$ fatty acid.

10. The use according to one of the claims 1 to 9, the organosilicone copolymers serving to improve the wet and dry combability of the hair.

11. The use according to one of the claims 1 to 9, **characterized in that** the organosilicone copolymers serve to increase the color intensity.

12. Agent for the oxidative dyeing of keratinic fibers, especially of human hair, containing, in addition to dye precursors, organosilicone copolymers as defined in one of the claims 1 to 9.

13. Method for dyeing keratinic fibers, especially human hair, **characterized in that** an agent of claim 12 is mixed immediately before use with an oxidizing agent preparation, the resulting application preparation being applied on the fibers and, after a period of action, being rinsed off once again.

14. Method for dyeing keratinic fibers, especially human hair, **characterized in that** an agent of claim 12 is applied on the fibers, after a period of action, a second preparation, which is obtained by mixing an agent of claim 12 with an oxidizing agent preparation immediately before use, is applied on the fibers and, after a period of action, the fibers are rinsed thoroughly.

15. Two-component kit for dyeing keratinic fibers, especially human hair, comprising a first preparation of claim 12, as well as a second preparation containing at least one oxidizing agent.

## Revendications

1. Utilisation de copolymères organosiliconés qui contiennent en tant que copolymères séquencés, des séquences polyoxyalkylène, des séquences polysiloxane, et au moins deux groupes amino liés de manière covalente à la chaîne principale du polymère ou au niveau des extrémités de chaîne, dans des agents pour la teinture par oxydation de fibres kératiniques, en particulier de cheveux humains.

2. Utilisation selon la revendication 1, **caractérisée en ce que** le copolymère d'organosiloxane est un copolymère séquencé statistique ou aléatoire, de séquences polyoxyalkylène et de séquences polysiloxane.

3. Utilisation selon la revendication 1 ou 2, **caractérisée en ce que** le copolymère organosiliconé contient des séquences polyoxyéthylène et polyoxypropylène et des séquences polydiméthylsiloxane.

4. Utilisation selon l'une quelconque des revendications 1 à 3, **caractérisée en ce que** le copolymère organosiliconé présente des groupes amino primaire et/ou amine secondaire.

5. Utilisation selon l'une quelconque des revendications 1 à 4, **caractérisée en ce que** le copolymère organosiliconé présente des motifs récurrents de formule générale (I)

$$[SiMe_2\text{-}O\text{-}(SiMe_2\text{-}O)_xSiMe_2\text{-}R\text{-}NH\text{-}R'\text{-}O\text{-}(C_2H_4O)_a\text{-}(C_3H_6O)_b\text{-}R'NH\text{-}R)] \qquad (I)$$

avec la signification :

x est un nombre de 3 à 500,
a est un nombre de 1 à 300,
b est un nombre de 0 à 300,
R représente indépendamment l'un de l'autre un radical organique bivalent, qui est incorporé dans la chaîne principale de polymère par l'intermédiaire de liaisons Si-C et C-N,
R' représente indépendamment l'un de l'autre un radical organique bivalent, qui est incorporé dans la chaîne principale de polymère par l'intermédiaire de liaisons C-O et C-N.

6. Utilisation selon l'une quelconque des revendications 4 ou 5, **caractérisée en ce que** le copolymère organosiliconé présente des groupes amino primaire en fin de chaîne.

7. Utilisation selon l'une quelconque des revendications 1 à 3, **caractérisée en ce que** le copolymère organosiliconé présente des groupes amino tertiaire.

8. Utilisation selon la revendication 7, **caractérisée en ce que** le copolymère organosiliconé présente des motifs récurrents de formule générale (II)

$$[(SiMe_2\text{-}O\text{-})_ySiMe_2\text{-}R^1\text{-}]_vNR^2[R^3\text{-}(OC_2H_4)_c\text{-}(OC_3H_6)_d\text{-}R^4\text{-}NR^5\text{-}]_w \quad (II)$$

avec la signification :

y est un nombre de 5 à 300,
v est un nombre de 1 à 50,
w est un nombre de 1 à 50,
c est un nombre de 5 à 200,
d est un nombre de 4 à 200,
$R^1$, $R^3$, $R^4$ représentent indépendamment l'un de l'autre des radicaux alkylène linéaires en $C_2$ à $C_{20}$, qui peuvent être substitués respectivement par un ou plusieurs groupes OH et être interrompus par un ou plusieurs groupes non voisins -O-, -C(O)-, -O-C(O), -C(O)-O-, et
$R^2$ et $R^5$ représentent indépendamment l'un de l'autre un groupe alkyle en $C_1$ à $C_4$.

**9.** Utilisation selon l'une quelconque des revendications 7 à 8, **caractérisée en ce que** le copolymère organosiliconé est mis à réagir avec un acide gras en $C_{16}$ à $C_{24}$.

**10.** Utilisation selon l'une quelconque des revendications 1 à 9, dans laquelle les copolymères organosiliconés servent à améliorer l'aptitude au coiffage des cheveux à l'état mouillé et sec.

**11.** Utilisation selon l'une quelconque des revendications 1 à 9, **caractérisée en ce que** les copolymères organosiliconés servent à augmenter l'intensité de la couleur.

**12.** Agent pour la teinture par oxydation de fibres kératiniques, en particulier de cheveux humains, contenant outre des produits précurseurs de colorant, des copolymères organosiliconés tels qu'ils sont définis selon l'une quelconque des revendications 1 à 9.

**13.** Procédé pour teindre les fibres kératiniques, en particulier les cheveux humains, **caractérisé en ce qu'**un agent selon la revendication 12 est mélangé directement avant l'application avec une préparation d'agent d'oxydation, la préparation d'application résultante étant appliquée sur les fibres est rincée de nouveau après une durée d'action.

**14.** Procédé pour teindre les fibres kératiniques, en particulier les cheveux humains, **caractérisé en ce qu'**un agent selon la revendication 12 est appliqué sur les fibres, après une durée d'action, une deuxième préparation, qui est obtenue directement avant l'application en mélangeant un agent selon la revendication 12 avec une préparation d'agent d'oxydation, est appliquée sur les fibres et, après une durée d'action, les fibres sont rincées minutieusement.

**15.** Nécessaire à deux composants pour teindre les fibres kératiniques, en particulier les cheveux humains, comprenant une première préparation selon la revendication 12 ainsi qu'une deuxième préparation, contenant au moins un agent d'oxydation.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 1356802 A
- WO 9909939 A
- WO 0141721 A
- WO 9732917 A
- DE 2359399
- JP 02019576 A
- WO 9615765 A
- DE 3843892 A
- DE 4133957 A
- WO 9408969 A
- WO 9408970 A
- EP 740931 A
- DE 19543988 A
- WO 9903819 A1
- WO 9903834 A2
- WO 9903836 A1
- WO 99148856 A1
- WO 99148874 A1
- WO 9948875 A1
- WO 0042971 A2
- WO 0042979 A1
- WO 00142980 A1
- WO 0043356 A1
- WO 0043367 A1
- WO 0043368 A1
- WO 0043386 A1
- WO 0043388 A1
- WO 0043389 A1
- WO 0043396 A1
- EP 0984006 A1
- EP 0984007 A1
- EP 0989128 A1
- EP 998908 A2
- WO 0121145 A


### In der Beschreibung aufgeführte Nicht-Patentliteratur


- *Eurocosmetics,* Marz 2002, 20-24
- Monographie Ch. Zviak. The Science of Hair Care. vol. 7, 248-250
- THE SCIENCE OF HAIR CARE. vol. 7, 264-267
- Dermatology. Verlag Marcel Dekker Inc, 1986
- **Kh. Schrader.** Grundlagen und Rezepturen der Kosmetika. Hüthig Buch Verlag, 1989